# FASCICULE DE BREVET EUROPEEN

(11) **EP 3 082 675 B1**
(45) Date de publication et mention de la délivrance du brevet: **27.03.2019**
(21) Numéro de dépôt: 14830667.3
(22) Date de dépôt: 16.12.2014
(51) Int. Cl.: A61F 13/00, A61F 13/02

(54) **ARTICLE POUR ABSORBER UN LIQUIDE PHYSIOLOGIQUE, NOTAMMENT PANSEMENT**
ARTIKEL ZUR ABSORPTION EINER PHYSIOLOGISCHEN FLÜSSIGKEIT, WIE ETWA EIN VERBAND
ARTICLE FOR ABSORBING A PHYSIOLOGICAL LIQUID, SUCH AS A DRESSING

(30) Priorité: 19.12.2013 FR 1363014
(43) Date de publication de la demande: 26.10.2016
(73) Titulaire: Commissariat à l'Énergie Atomique et aux Énergies Alternatives, 75015 Paris (FR); Urgo Recherche Innovation et Développement, 21300 Chenove (FR)
(72) Inventeur: REVOL-CAVALIER, Frédéric, F-38180 Seyssins (FR); LAMOISE, Michel, F-21110 Bessey Les Citeaux (FR); MARSIQUET, Cyril, F-16270 Roumazières Laubert (FR); PERNOT, Jean-Marc, F-21000 Dijon (FR); STEINBRUNN, Julien, F-21380 Messigny et Vantoux (FR)
(74) Mandataire: Nony
(86) Numéro de dépôt international: PCT/IB2014/066949
(87) Numéro de publication internationale: WO 2015/092670

(56) Documents cités:
- WO-A1-00/42958
- WO-A1-2014/016759
- DE-U1-202012 101 743
- US-A1- 2003 088 229
- US-A1- 2012 136 326

## Description

La présente invention concerne des articles destinés à venir en contact avec des liquides physiologiques, en particulier des liquides sécrétés par la peau, une plaie et/ou des muqueuses, pour gérer la propagation, le stockage et/ou l'évaporation de ces liquides et, plus particulièrement mais non exclusivement, des pansements à appliquer sur une plaie.

La gestion de la propagation des liquides ainsi que leur stockage sont des problèmes complexes à résoudre pour lesquels les articles proposés dans le domaine des pansements et des produits d'hygiène ne fournissent pas aujourd'hui des solutions entièrement satisfaisantes.

Ces articles doivent en effet satisfaire à un cahier des charges qui comprend des exigences antagonistes.

Une première exigence est d'éloigner le plus loin et le plus rapidement possible les liquides afin d'éviter à cause de leur accumulation tout phénomène de macération ou d'irritation au niveau de la peau, de la plaie ou des muqueuses. Non seulement les liquides ne doivent pas s'accumuler, mais il est aussi préférable d'éviter leur migration latérale à partir du lieu de sécrétion, pour ne pas augmenter l'étendue de la zone humidifiée, à des fins de garantir une meilleure hygiène et contribuer au confort de l'utilisateur.

Ceci est particulièrement important lors de l'utilisation d'un pansement. Dans ce cas, il est primordial d'éviter que la peau située en bordure de la lésion, appelée peau périlésionnelle, qui est très fragile, soit humide, car cela peut provoquer son altération, favorisant par exemple une infection et/ou une irritation.

Ainsi, il est souhaitable que le pansement draine efficacement les liquides corporels sécrétés par la plaie depuis leur lieu de sécrétion vers la périphérie de ce lieu. Un tel drainage assure une meilleure hygiène et des conditions de cicatrisation améliorées.

Une seconde exigence est de stocker ces liquides et d'éviter leur retour vers la peau, la plaie ou les muqueuses. Un stockage important permet d'augmenter la durée d'utilisation de l'article. Dans le cas d'un pansement, cette durée d'utilisation est particulièrement importante car elle permet de diminuer le risque d'altérer le processus de cicatrisation de la plaie en changeant ce dernier moins souvent.

Il est donc souhaitable de disposer d'articles qui permettent de gérer la propagation et le stockage des liquides, en particulier des liquides sécrétés par une plaie, la peau ou les muqueuses, en éloignant ces derniers de leur point de sécrétion pour éviter leur accumulation et accroître la durée d'utilisation de l'article.

Pour rester le plus longtemps possible sur une plaie, le pansement doit permettre un drainage important des liquides. Ce drainage est la somme de sa capacité d'absorption et de sa capacité à éliminer les liquides stockés dans une couche absorbante au contact de la plaie, par évaporation de la vapeur d'eau à travers un support protecteur du pansement, qui protège la plaie et la couche absorbante de l'environnement extérieur (bactéries, eau, ...).

US2003088229 décrit un article absorbant comportant une couche d'acquisition venant en contact avec la surface de la peau présentant des fils orientés selon l'axe longitudinal du pansement et séparés par des espaces libres permettant le stockage temporaire du fluide avant son absorption par une couche de stockage. De tels fils permettent une redistribution rapide des fluides dans la couche de stockage.

EP 541391 propose d'incorporer entre la couche absorbante et le support une couche qui favorise l'étalement des liquides pour optimiser leur évaporation. Toutefois, cette solution est loin d'être optimale, car quand la couche d'étalement est saturée d'eau l'évaporation devient moins effective et surtout les liquides ont tendance à redescendre dans les parties de la couche absorbante non encore en contact avec les liquides. On retrouve alors les risques liés à l'accumulation des liquides au niveau de la plaie et de la peau périlésionnelle, comme par exemple la macération et les possibilités d'infection. On diminue aussi la capacité d'absorption de la couche absorbante, voire on entraine sa déformation, suite à la création de gradients de teneur en liquide en son sein.

Pour éviter ces problèmes et augmenter le drainage, il a donc été proposé de jouer sur la capacité d'absorption du pansement en superposant plusieurs couches absorbantes. Cette solution présente l'avantage, en jouant sur les différences d'absorption de deux couches ou l'épaisseur de la première couche qui vient au contact de la plaie, d'éloigner les liquides de la plaie et d'éviter les problèmes de macération ou d'infection liés à leur présence au niveau de la plaie et de la peau périlésionnelle.

Pour augmenter cet effet de « pompage » des liquides, il a été proposé d'incorporer entre la couche absorbante au contact de la plaie et la couche absorbante qui lui est superposée, et qui sert de réservoir, une couche d'étalement. Un tel pansement a par exemple été commercialisé par la société MOLNLYCKE Health Care au début des années 2000 sous la dénomination Mepilex Border.

Pour optimiser le drainage de ce pansement, la demande de brevet WO 2010/147533 a proposé d'ajouter une nouvelle couche d'étalement des liquides entre la couche réservoir et le support.

Mais les solutions proposées restent encore loin d'être complétement satisfaisantes. L'augmentation du nombre de couches superposées conduit à des pansements plus épais dans lesquels les risques de fuites sont augmentés. Or, il est souhaitable d'avoir des produits les plus minces et les plus souples et conformables possibles pour s'adapter facilement à l'anatomie de la zone du corps sur laquelle ils sont appliqués.

Afin de répondre à cette problématique d'adaptation, DE 20 2012 101743 propose un pansement présentant des incisions ou des régions découpées permettant de faciliter l'application du pansement sur des surfaces à topologies convexes ou irrégulières.

L'optimisation des capacités de stockage conduit aussi, en raison de l'accumulation des liquides, à des articles plus lourds pour lesquels les risques de détachement à cause du poids dans le cas d'un pansement adhésif, par exemple à cause du positionnement vertical du pansement sur un ulcère de jambe ou une partie non plane du corps, sont augmentés. Pour contrecarrer ce défaut, il faudrait augmenter le pouvoir d'adhésion de l'adhésif. Mais dans un pansement augmenter le pouvoir d'adhésion est toujours difficile, car il faut trouver un équilibre entre un pouvoir adhésif élevé pour garantir l'absence de décollement et un pouvoir adhésif suffisamment faible pour ne pas altérer la peau périlésionnelle et le processus de cicatrisation de la plaie quand le pansement est retiré.

De plus, la présence de la couche réservoir diminue la perméabilité à la vapeur d'eau globale du pansement et ceci même si l'on ajoute une couche d'étalement entre le support et la couche réservoir.

Enfin, si une telle structure de pansement permet d'éloigner les liquides au niveau de la plaie, cette augmentation de la capacité de « pompage » des liquides peut entrainer, quand le niveau d'exsudation de la plaie diminue ou si le pansement est utilisé sur une plaie moins exsudative, un assèchement du lit de la plaie, ce qui est défavorable au processus de cicatrisation.

Ainsi, bien que depuis le début des années 1990 l'utilisation d'une couche d'étalement soit envisagée pour favoriser le drainage d'un pansement, la mise au point d'un produit entièrement satisfaisant reste encore à réaliser.

Par ailleurs, la présence d'une couche d'étalement, constituée d'une bande drainante, permet de repartir le liquide sur toute la surface de cette couche afin de le transporter rapidement d'une zone de captation vers une zone de stockage. Par principe, la surface de la couche d'étalement est rapidement mouillée, ce qui est un avantage si l'on veut répartir ou drainer rapidement le liquide, mais cela peut être également un inconvénient si cette couche recouvre des parties du pansement qui ne doive pas être mouillées. En effet, la couche d'étalement peut redistribuer le liquide qu'elle transporte sur des parties sèches du pansement avant de les transférer vers la zone de stockage.

US 2011/0077571 A1 divulgue un pansement comportant deux bandes drainantes d'un matériau absorbant qui conduisent respectivement d'une couche absorbante placée au-dessus de la plaie à deux réservoirs déportés. Les deux bandes se superposent sans interposition d'une barrière étanche entre elles, ce qui permet un passage de liquide d'une bande à l'autre en présence d'un contact entre elles. Cela entraîne un risque de retour du liquide provenant de l'une des bandes vers une zone encore sèche de la couche absorbante, par l'intermédiaire de l'autre bande.

Il est souhaitable de disposer d'un pansement dont la structure permette d'éviter l'accumulation des liquides au niveau de la plaie et autour de celle-ci, tout en présentant une bonne perméabilité à la vapeur d'eau et qui soit mince, conformable et utilisable quel que soit le niveau d'exsudation de la plaie.

L'invention vise à réaliser des articles, en particulier des pansements, qui répondent à l'objectif énoncé ci-dessus. Ces pansements sont conditionnés à l'état stérile, de préférence.

L'invention cherche également à perfectionner des articles d'hygiène autres que des pansements, tels que des couches-culottes ou articles d'hygiène féminine, en offrant une vitesse d'absorption des fuites corporelles élevée, sans nuire au confort de l'utilisateur.

L'invention a pour objet un article ayant des propriétés d'absorption d'un fluide corporel, comprenant au moins trois chemins de propagation fluidique depuis une zone de captation dudit fluide, vers au moins une zone de stockage et/ou d'évaporation, chaque chemin de propagation fluidique étant délimité par une barrière fluidique, s'opposant au passage de liquide d'un chemin à un autre.

L'article peut être configuré pour absorber un exsudat, émis par une plaie, et constituer un pansement.

L'invention permet de réduire le risque d'une propagation du liquide provenant de la zone de captation ailleurs que vers la zone de stockage et/ou d'évaporation, et celui de mouiller une partie sèche de l'article au contact de la peau ailleurs que dans la zone de captation.

Dans l'invention, le liquide qui diffuse préférentiellement au sein d'un premier chemin de propagation fluidique ne gagne pas ou peu le ou les chemins de propagation fluidique adjacents. On évite ainsi, lorsque ceux-ci n'ont pas encore été sollicités et sont secs, par exemple, qu'ils n'absorbent une certaine quantité de liquide au détriment de la zone de stockage et/ou d'évaporation vers laquelle conduit le premier chemin de propagation fluidique.

A cet effet, la barrière fluidique selon l'invention comporte un espace libre et/ou un matériau hydrophobe. La barrière fluidique peut s'étendre entre deux chemins de propagation fluidiques adjacents.

L'invention peut permettre de réduire l'étendue de la surface de l'article mouillée par le liquide, ailleurs que dans la zone de captation et de la zone de stockage et/ou d'évaporation. Le confort apporté par l'article est amélioré, et dans le cas d'un pansement, cela peut permettre de le laisser plus longtemps en place.

L'invention permet d'optimiser le transfert d'une partie du fluide absorbé par l'article vers une ou plusieurs parties absorbantes définissant la zone de stockage et/ou d'évaporation, encore appelée(s) réservoir(s). Cette ou ces parties absorbantes peuvent être juxtaposées à la zone de captation ou bien déportées afin de faciliter leur éventuel remplacement.

Selon l'invention, le transfert du liquide s'effectue avec un débit plus important le long d'un chemin de propagation fluidique que d'un chemin à l'autre. Cela peut être obtenu en utilisant pour réaliser les chemins de propagation fluidique des éléments d'une structure de transfert disjoints, de telle sorte que le liquide ne peut pas diffuser d'un élément à l'autre faute d'une continuité de matière absorbante entre eux.

Une autre façon est de réaliser les éléments de la structure de transfert au sein d'un matériau qui présente des propriétés de drainage du liquide différentes selon la position sur le matériau. Par exemple, le matériau est hydrophile le long des chemins de propagation fluidique et hydrophobe entre lesdits chemins, de telle sorte que le passage du liquide d'un chemin à l'autre est contrarié. Les chemins peuvent être formés en traitant localement un matériau poreux hydrophile de façon à définir des zones hydrophobes s'étendant entre les zones hydrophiles, ces dernières définissant les chemins de propagation fluidique. En variante, les chemins sont formés en traitant localement un matériau poreux hydrophobe de façon à définir des zones hydrophiles constituant les chemins de propagation fluidique.

Le blocage de la diffusion du liquide d'un chemin à l'autre peut être total, notamment dans le cas d'éléments disjoints, ou n'être que partiel, par exemple dans le cas de propriétés localement différentes du matériau définissant des chemins de propagation.

Le liquide drainé par les chemins de propagation fluidique peut être transféré vers la zone de stockage et/ou d'évaporation et non pas redistribué en partie sur l'article pendant son transport, ce qui constitue un premier avantage de l'invention, comme indiqué ci-dessus.

Un autre avantage est que la structure de transfert qui définit ces chemins peut ne pas recouvrir totalement l'article, ce qui augmente sa respirabilité, en particulier lorsque ces chemins sont définis par des éléments disjoints. Par éléments disjoints, on entend des éléments séparés l'un de l'autre par un espace libre. Le terme libre désigne l'absence de matériau. En particulier, l'espace libre peut être comblé d'air.

Les chemins de propagation fluidique peuvent être disposés de façon sensiblement parallèle les uns aux autres et/ou avec une imbrication éventuelle des chemins entre eux. Les chemins peuvent encore être disposés avec un décalage angulaire autour d'un centre de l'article, de préférence en étant alors équiangulairement répartis.

Dans le cas où les chemins sont disposés de façon sensiblement parallèle, ceux-ci peuvent définir deux ensembles de chemins de propagation fluidique entre une zone de captation commune de fluide et deux zones de stockage et/ou d'évaporation respectives, chaque zone de stockage et/ou d'évaporation étant propre à l'un des deux ensembles, les chemins s'interpénétrant au niveau de la zone de captation commune.

Dans le cas où les chemins sont disposés avec un décalage angulaire, les chemins de propagation fluidique peuvent s'étendre depuis une zone de captation commune de fluide vers une zone de stockage et/ou d'évaporation commune, de sorte que la zone de stockage et/ou d'évaporation entoure complètement la zone de captation, les deux zones étant par exemple concentriques. En variante, chaque chemin peut drainer le liquide vers une zone de stockage et/ou d'évaporation qui lui est propre.

La zone de captation du fluide est de préférence adaptée à la forme de la source du fluide. Par exemple, dans le cas d'une plaie faisant suite à une entaille ou une coupure, la zone de captation peut être de forme allongée. Dans le cas d'un article à appliquer sur une partie du corps non plane, la zone de captation peut épouser le relief de la partie du corps en question.

Le nombre de chemins de propagation fluidique est par exemple compris entre 3 et 10, à 50, voire 100. Ce nombre dépend notamment de la surface de la zone de captation.

Les chemins de propagation fluidique peuvent être définis par des éléments se superposant au moins partiellement afin d'optimiser la surface de l'article. Un matériau imperméable à l'eau peut alors limiter, mieux empêcher, le transfert de liquide depuis un élément vers un autre élément lui étant superposé.

Dans une autre variante, les chemins de propagation fluidique sont définis par des éléments dont chacune des deux extrémités débouche sur une partie absorbante formant zone de stockage et/ou d'évaporation. La zone de captation du fluide est alors située entre les deux parties absorbantes formant zones de stockage et/ou d'évaporation, au contact d'une partie de la face inférieure des éléments.

La zone de captation de l'article est définie par une partie absorbante réceptrice. La zone de stockage et/ou d'évaporation de l'article est définie par au moins une partie absorbante formant zone de stockage et/ou d'évaporation.

### Partie absorbante réceptrice et partie absorbante formant zone de stockage et/ou d'évaporation

La partie absorbante réceptrice est encore qualifiée de « couche absorbante » et la partie absorbante formant zone de stockage et/ou d'évaporation de « couche formant réservoir » ou « réservoir ». Le terme « couche » doit se comprendre comme englobant un agencement monocouche ou plusieurs sous-couches assemblées.

La partie absorbante réceptrice et la partie absorbante formant zone de stockage et/ou d'évaporation présentent chacune de préférence une capacité d'absorption d'eau supérieure ou égale à 500 g/m², mieux à 800g/m². Elles peuvent comporter ou être constituées de tout matériau apte à stocker les liquides, comme par exemple les matériaux utilisés dans le domaine de l'hygiène et des pansements.

La partie absorbante formant zone de stockage et/ou d'évaporation est définie par un matériau ayant une capacité d'absorption du liquide supérieure à celle de la structure de transfert. Il en est de même de la partie absorbante réceptrice. Ainsi, la capacité d'absorption d'eau ; en g/m², de la structure de transfert est inférieure à celle de la partie absorbante réceptrice et à celle de la partie absorbante formant zone de stockage et/ou d'évaporation.

On peut citer, à titre d'exemple, les mousses absorbantes et de préférence les mousses de polyuréthane hydrophiles, tous les matériaux à base de polymère superabsorbant (SAP), comme par exemple les non tissés absorbants incorporant des particules de SAP couramment utilisés dans le domaine de l'hygiène, les textiles absorbants comme par exemple les non tissés à base de viscose, de rayonne ou de cellulose, tels que par exemple une ouate, ou des hydrogels.

On préfère, dans le cadre de la présente invention, utiliser comme partie absorbante réceptrice un matériau alvéolaire, par exemple une mousse de polyuréthane hydrophile, à l'image par exemple de celle commercialisée sous la dénomination MCF.03 par la société Advanced Medical Solution.

Pour la partie absorbante formant zone de stockage et/ou d'évaporation, on préfère l'utilisation d'un matériau comportant un polymère superabsorbant SAP.

On préfère l'utilisation de non tissés obtenus par la méthode de fabrication par voie sèche, connue sous le nom de voie aérodynamique ou « airlaid », qui contiennent des particules de SAP et en particulier entre 20 et 60 % en poids de SAP par rapport au poids total du non tissé. De tels non tissés sont par exemple commercialisés par la société EAM Corporation sous la référence Novathin®.

Selon un mode préféré de mise en oeuvre de l'invention, on utilise pour réaliser la partie absorbante formant zone de stockage et/ou d'évaporation un non tissé à base de particules de polymères superabsorbants et de fibres de cellulose sans incorporation de matériaux thermoliants ou de latex, et qui est recouvert sur chacune de ses faces par un voile cellulosique.

Selon une variante, on emploie comme matériau à base de SAP un matériau constitué de deux voiles cellulosiques entre lesquels sont incorporées des particules de polymères superabsorbants, seules ou en association avec des liants.

Selon une autre variante, on utilise un matériau à base de fibres de SAP seules ou en association avec des fibres non absorbantes. De préférence, ce matériau se présente sous forme d'un non tissé.

Afin de s'adapter au mieux aux plaies usuelles, notamment les plaies résultant de coupures ou entailles, la partie absorbante réceptrice peut être de forme allongée.

De préférence, la partie absorbante réceptrice est formée d'une couche absorbante recouverte d'une couche d'interface au contact de la plaie, qui évite d'altérer le processus de cicatrisation de la plaie lors du retrait du pansement. Cette couche d'interface est de préférence recouverte d'un voile protecteur provisoire, qui est retiré avant usage.

On peut utiliser pour une telle couche des pansements connus sous le nom de « pansements interface », comme par exemple les produits commercialisés par les sociétés Laboratoires URGO et MOLNLYCKE HEALTH CARE respectivement sous les dénominations URGOTUL® et MEPITEL®.

On peut aussi utiliser des couches perforées de formulation hydrophobe ou hydrophile, mais non absorbantes ou peu absorbantes, à base de polymères. Ces formulations peuvent être adhérentes ou non adhérentes.

On préfère utiliser des formulations microadhèrentes ou non adhérentes. De telles formulations sont bien connues de l'homme de l'art et sont par exemple réalisées à base de gel(s) de silicone, d'adhésif(s) siliconé(s) sensibles à la pression ou de compositions contenant un élastomère séquencé du type poly (styrène -oléfine - styrène), un plastifiant tel une huile minérale et une faible quantité d'hydrocolloïde(s) pour créer un environnement humide favorisant le processus de cicatrisation sans rendre la composition absorbante pour éviter de boucher les trous. De telles formulations microadhérentes sont par exemple utilisées dans les pansements commercialisés par la société Laboratoires URGO sous les dénominations URGOCLEAN® et URGOTUL ABSORB®.

Selon des variantes possibles, ces pansements interfaces et ces couches perforées de formulation hydrophobe ou hydrophile sont associés à des matériaux perméables, en particulier des non tissés non absorbants, qui sont incorporés entre la couche absorbante et les couches supplémentaires.

Comme mentionné plus haut, les chemins de propagation fluidique depuis la zone de captation vers la zone de stockage et/ou d'évaporation sont définis par une structure de transfert.

### Structure de transfert

La structure de transfert est encore appelée « couche de distribution ».

Son rôle n'est pas de stocker les liquides mais de permettre leur étalement.

La structure de transfert est moins absorbante d'une part que la partie absorbante réceptrice et d'autre part que la partie absorbante formant zone de stockage et/ou d'évaporation. La structure de transfert présente de préférence une valeur d'absorption maximale qui est inférieure à 500 voire1000 g/m².

De préférence, son épaisseur est comprise entre 50 et 1000 µm, mieux entre 300 et 500 µm.

La plus grande dimension de la structure de transfert est par exemple supérieure ou égale à 10 cm, voire 30 cm, la plus grande dimension étant alors la longueur.

La structure de transfert comporte ou est constituée d'un matériau fibreux, à base de fibres absorbantes, de façon à permettre un drainage efficace du liquide. Dans ce type de matériau, le liquide se propageant, de proche en proche, le long des fibres, ce qui produit un effet de drainage.

A titre d'exemple de tels matériaux, on peut citer les matériaux à base de fibres absorbantes d'origine végétale, telle que la viscose, la cellulose ou ses dérivés. Ces matériaux peuvent se présenter sous forme de tricots, de tissés ou de non tissés, obtenus par voie sèche ou par voie humide, comme les papiers.

Dans le cadre de la présente invention, on préfère les non tissés et les papiers. Parmi les non tissés, on préfère ceux à base de fibres absorbantes, telles que la viscose ou la cellulose, associées à des fibres non absorbantes, comme par exemple des fibres de polyester ou de polyoléfine. A titre d'exemple de tels non tissés, on peut citer les produits commercialisés respectivement par les sociétés Suominen Corp et Orsa sous les dénominations Fibrella® 2000 et Jettex® 1205 c, ou Berkshire sous la dénomination DR870.

La structure de transfert peut se présenter sous la forme d'au moins trois éléments disjoints, directement en contact avec une couche d'un matériau absorbant de la partie absorbante réceptrice, sans couche intermédiaire entre les deux. En variante, une couche intermédiaire peut s'interposer, par exemple une couche ajourée d'un matériau hydrophobe, de façon à réduire le risque de retour de liquide vers la partie absorbante réceptrice. En variante, la zone de captation peut être définie par une portion de la structure de transfert directement au contact de la plaie, ou avec interposition d'un pansement interface.

La structure de transfert peut présenter des éléments définissant les chemins de propagation fluidique qui sont plus rapprochés les uns des autres au niveau de la zone de captation. Ces éléments peuvent être dans un même plan ou s'adapter au relief de la zone du corps ou à la forme de la plaie, d'où provient le liquide physiologique à capter.

Lorsque la structure de transfert présente des éléments disjoints qui définissent des chemins de propagation fluidique respectifs, ces éléments peuvent être découpés dans une feuille d'un même matériau hydrophile, comportant de préférence des fibres absorbantes. Ces éléments peuvent être maintenus dans une configuration géométrique donnée par une ou plusieurs couches adjacentes de l'article ou par tout autre moyen adapté. Le cas échéant, les éléments sont assemblés les uns aux autres avec interposition de couches barrières bloquant au moins partiellement, mieux totalement, la diffusion du liquide d'un élément à l'autre.

Une autre façon de réaliser la structure de transfert est d'appliquer un traitement différencié à un même matériau de départ, en feuille. Par exemple, un matériau absorbant hydrophile tel qu'un papier est traité en déposant un matériau hydrophobe, par exemple une résine silicone, appliquée selon une structuration, notamment sous la forme de lignes, de préférence continues, délimitant entre elles des chemins de propagation fluidique. Chaque ligne de matériau hydrophobe constitue alors une barrière fluidique, bloquant le passage du fluide d'un chemin à un autre.

### Support

L'article peut comporter un support, qui peut définir une partie au moins de la surface extérieure de l'article.

Le support est de préférence imperméable à l'eau et aux micro-organismes pathogènes extérieurs, tout en assurant une perméabilité à la vapeur d'eau, de manière à éviter à la fois le contact de la plaie avec des liquides extérieurs et des bactéries et la macération de la plaie. Le support est alors qualifié de « imperméable et respirant ».

Le support est de préférence mince et souple, de manière à mieux épouser la forme du corps et suivre les mouvements de celui-ci sans risquer de se détacher. Le support est avantageusement conformable. Son épaisseur peut être comprise entre 100 et 600 µm, de préférence entre 250 et 500 µm.

Le support peut être constitué d'un seul matériau ou de l'assemblage de plusieurs matériaux.

Le support peut ainsi comporter ou être constitué d'un film continu et imperméable aux liquides et aux bactéries mais perméable à la vapeur d'eau. Parmi les films utilisables, on peut citer à titre d'exemple les films en polyétheruréthane, en polyétheramide, ou en polyétherester. L'épaisseur du film est par exemple comprise entre 5 et 200 microns, de préférence entre 10 et 75 microns, de préférence encore entre 10 et 50 microns.

Le film présente avantageusement un taux de transmission de vapeur d'eau (MVTR : Moisture Vapor Transmission Rate) supérieur à 3 000 g/m²/24 heures, de préférence supérieur ou égal à 7 000 g/m²/24 heures, de préférence encore supérieur ou égal à 10 000 g/m²/24 heures. Une technique de mesure du taux de transmission de vapeur d'eau en contact liquide est décrite dans la norme NF-EN 13726-2 (Chapitre 3.3).

De tels films sont couramment utilisés dans la réalisation de pansements et sont par exemple constitués de films de polyuréthane, tels les films commercialisés par la société Exopack Advanced Coating sous la désignation INSPIRE.

Le film peut être remplacé par un complexe mousse/film. Le film peut aussi être complexé avec un autre matériau qui sert alors d'armature pour rigidifier le support.

Une telle armature peut permettre de rigidifier le support, de manière à ce qu'il ne s'enroule pas sur lui-même après retrait d'un voile protecteur pelable éventuel.

L'armature peut être constituée de tout matériau ajouré, tel un film perforé, un filet thermoplastique, un textile comme par exemple un tissé, un tricot ou un non tissé, de préférence élastique, pour une meilleure tenue de l'article sur la peau.

Lorsqu'un film perforé est utilisé pour réaliser l'armature, celui-ci est par exemple en polyéthylène ou en polypropylène. Lorsqu'un textile tissé est utilisé, celui-ci est par exemple en polyéthylène téréphtalate ou en polyamide. Le grammage de l'armature est de préférence compris entre 10 et 500 g/m², par exemple entre 20 et 300 g/m². Des pansements avec de tels supports avec armature sont décrits dans la demande WO 2012/140377.

Le film ou le complexe servant de support peut être assemblé aux autres couches du pansement à l'aide d'un adhésif discontinu, de manière à ne pas affecter la perméabilité du film ou du complexe à la vapeur d'eau.

Le support peut être utilisé pour assurer le maintien des éléments de la structure de transfert dans la disposition recherchée ; le liquide qui diffuse dans les éléments ne peut pas diffuser dans le support à leur contact, qui est imperméable, pour passer d'un élément à l'élément voisin.

L'invention a encore pour objet l'utilisation de l'article selon l'invention pour absorber un liquide physiologique autre que l'exsudat d'une plaie. Ainsi, dans des exemples de mise en oeuvre de l'invention, celle-ci s'applique à la réalisation de couches-culottes et d'articles d'hygiène féminine.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente de façon schématique et partielle, en coupe, un exemple d'article réalisé conformément à l'invention,
- la figure 2 représente isolément, en vue de dessus, la structure de transfert de l'exemple de la figure 1,
- la figure 3 illustre une variante de réalisation de la structure de transfert,
- les figures 4 et 5 sont des vues analogues à la figure 2, de variantes d'articles réalisés conformément à l'invention,
- la figure 6 est une vue analogue à la figure 2, d'une variante d'article,
- la figure 7 est une vue schématique et partielle, en perspective, d'une autre variante de réalisation d'article selon l'invention,
- la figure 8 est une vue schématique, en perspective, partielle, d'un autre exemple de réalisation de la structure de transfert, et
- les figures 9 et 10 sont des vues de dessus, schématiques et partielles, de variantes d'articles.

Sur le dessin, les proportions réelles n'ont pas toujours été respectées, dans un souci de clarté. De même, certains éléments peuvent avoir été représentés avec un léger espacement entre eux sur le dessin alors qu'ils sont en contact dans la réalité.

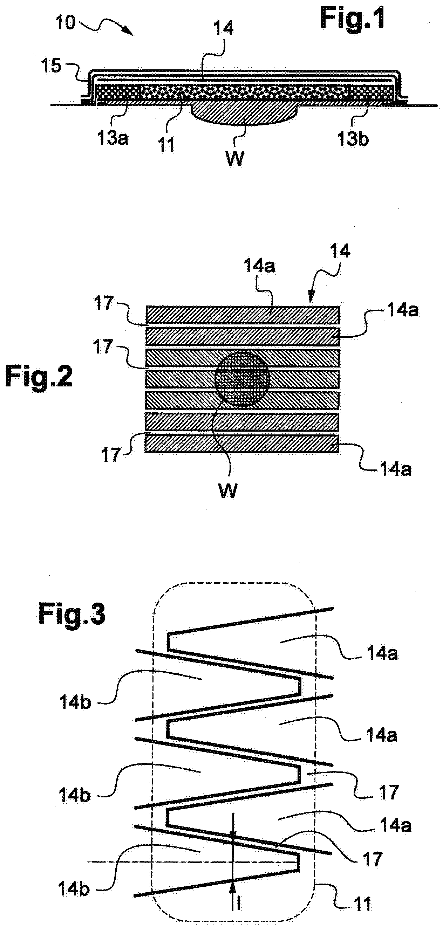

L'article 10 représenté à la figure 1 est destiné à être appliqué sur une partie du corps humain ou animal, pour absorber un liquide physiologique tel qu'un exsudat. Il s'agit de préférence d'un pansement, destiné à être appliqué sur une plaie W.

L'article 10 comporte, comme dans l'exemple illustré, une partie absorbante réceptrice 11, destinée à recevoir l'exsudat, définissant une zone de captation 12 de l'exsudat, et au moins une partie absorbante formant zone de stockage et/ou d'évaporation, reliée à la partie absorbante réceptrice 11 par une structure de transfert 14 selon l'invention.

Afin de réduire le risque de retour d'exsudat vers la partie absorbante réceptrice 11, l'article 10 peut comporter une couche intermédiaire telle que définie précédemment, non représentée sur le dessin, située entre la structure de transfert 14 et la partie absorbante réceptrice 11.

Dans l'exemple illustré, l'article 10 comporte deux parties absorbantes 13a et 13b formant zone de stockage et/ou d'évaporation, qui sont disposées respectivement de part et d'autre de la partie absorbante réceptrice 11.

L'article 10 peut également comporter un support 15 constitué d'une couche imperméable à l'eau, pouvant comme illustré recouvrir supérieurement les parties 13a et 13b formant zone de stockage et/ou d'évaporation et la structure de transfert 14.

La partie absorbante réceptrice 11, la structure de transfert 14 et le support 15 sont tels que définis de façon générale plus haut.

La structure de transfert 14 présente des propriétés drainantes, permettant un transfert par diffusion du liquide depuis la partie absorbante réceptrice 11 vers les parties absorbantes 13a et 13b formant zone de stockage et/ou d'évaporation.

La structure de transfert 14 comporte plusieurs éléments de transfert 14a comme illustré sur la figure 2. Ces éléments 14a sont dans l'exemple considéré sous forme de bandes sensiblement parallèles entre elles et espacées les unes des autres par un intervalle 17.

Ainsi, le liquide peut diffuser dans chaque élément en bande 14a sans diffuser d'un élément 14a à un élément voisin 14a, du fait de la présence de l'intervalle 17 entre les deux. L'intervalle 17 est un espace libre, formant une barrière fluidique entre deux éléments 14a adjacents.

Les éléments 14a permettent de définir une direction de drainage qui leur est parallèle, et le liquide peut ainsi être drainé depuis la plaie W vers les parties absorbantes 13a et 13b entre lesquelles s'étendent les éléments 14a.

Sur la figure 2, on voit que seuls trois éléments 14a situés au-dessus de la plaie W sont mouillés par l'exsudat, les autres éléments 14a restant secs.

Le fait d'éviter une migration latérale du fluide drainé évite ainsi de mouiller la partie de la structure de transfert 14 qui n'est pas en contact avec le fluide corporel.

Dans le cas d'un pansement, on empêche donc la peau périlésionnelle d'être en contact avec l'exsudat. Dans le cas d'article d'hygiène féminine ou de couche-culotte, on réduit le risque de mouiller les parties du corps sèches, proches de la source du fluide corporel.

Les éléments de la structure de transfert 14 comportent des fibres absorbantes et offrent au transport de l'exsudat une section transversale croissante en direction de la partie formant zone de stockage et/ou d'évaporation, comme illustré à la figure 3.

Plus particulièrement, les éléments de la structure de transfert 14 peuvent présenter en vue de dessus une forme générale trapézoïdale, dont la largeur *l* croît de façon linéaire lorsque l'on se rapproche de la partie absorbante formant zone de stockage et/ou d'évaporation. Cette augmentation de la largeur *l* permet d'accroître la vitesse de diffusion du liquide, donc la quantité de liquide collectée par la partie absorbante formant zone de stockage et/ou d'évaporation, ce qui permet d'améliorer la performance de l'article dans la captation d'exsudat.

Le fait que chaque élément présente une largeur croissante, mesurée perpendiculairement à son axe longitudinal, peut aussi faciliter l'imbrication des éléments et donne la possibilité d'avoir des éléments de transfert nombreux et rapprochés, bien que disjoints, au-dessus de la partie absorbante réceptrice 11, comme illustré à la figure 3. Par ailleurs, on a constaté qu'un élément de transfert ayant une largeur croissante permet un drainage optimisé d'un fluide.

On voit sur cette figure que l'on peut réaliser la structure de transfert 14 avec deux ensembles d'éléments 14a et 14b qui s'imbriquent au niveau de la zone de captation, les éléments d'un même ensemble définissant chacun un chemin de propagation fluidique.

Un intervalle 17 entre les éléments 14a et 14b d'une part et entre les éléments 14a ou 14b d'autre part, permet d'éviter la migration du liquide d'un élément 14a à un élément 14b ou entre deux éléments 14a ou 14b.

Dans une variante, les éléments 14a se raccordent entre eux à une extrémité, qui est superposée à la partie absorbante formant zone de stockage et/ou d'évaporation 13a. Il en est de même des éléments 14b.

La structure de transfert 14 peut présenter une épaisseur sensiblement constante sur toute sa longueur. L'épaisseur peut encore varier.

Les éléments de transfert 14a sont par exemple d'axes longitudinaux Y parallèles entre eux, de même que les éléments 14b, comme illustré, mais peuvent aussi avoir des orientations différentes.

Les éléments 14a, 14b sont par exemple découpés d'une seule pièce dans une feuille d'un papier ou non tissé comportant des fibres absorbantes comme détaillé plus haut.

Les parties absorbantes formant zones de stockage et/ou d'évaporation 13a et 13b peuvent être disjointes, comme illustré à la figure 2. En variante, celles-ci se rejoignent, et entourent par exemple complètement la partie absorbante réceptrice 11.

L'assemblage entre la partie absorbante réceptrice 11 et la structure de transfert 14 peut s'effectuer à l'aide d'un adhésif qui est par exemple appliqué de façon discontinue. Il en est de même de l'assemblage entre la structure de transfert 14 et la ou chaque partie absorbante formant zone de stockage et/ou d'évaporation.

La structure de transfert 14 peut être utilisée pour évaporer le liquide dont elle se charge, et le dispositif peut être réalisé sans partie absorbante formant zone de stockage et/ou d'évaporation en communication fluidique avec la structure de transfert.

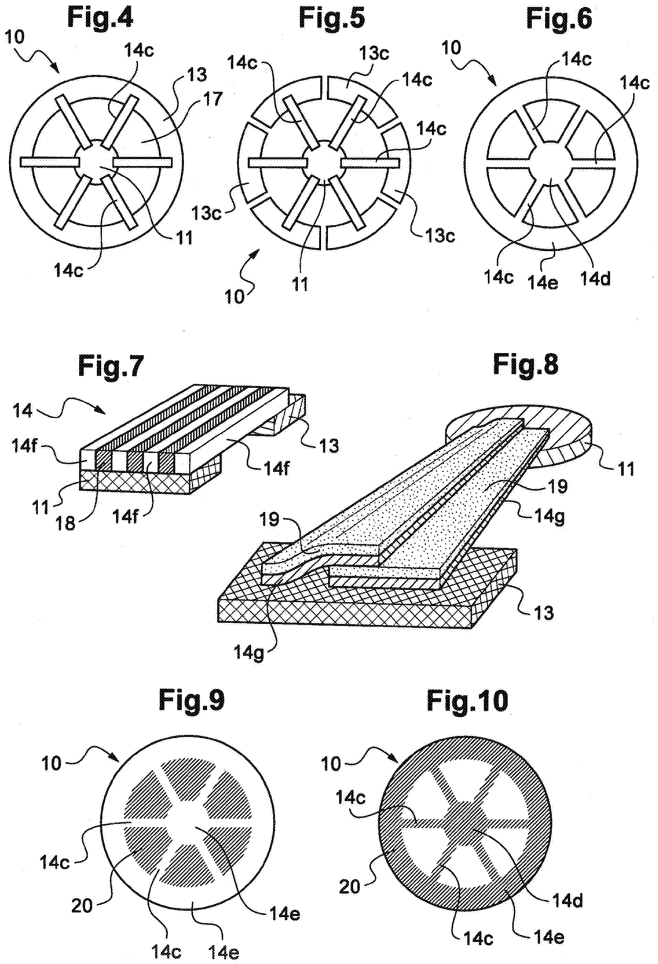

On a représenté à la figure 4 une variante de réalisation dans laquelle la structure de transfert 14 comporte des éléments de transfert radiaux 14c, s'étendant depuis une partie absorbante réceptrice centrale jusqu'à une partie absorbante 13 formant zone de stockage et/ou d'évaporation, périphérique, et de forme continue annulaire autour de la zone de captation 11.

Les éléments 14c sont par exemple disposés de façon équiangulairement répartie autour de la partie absorbante réceptrice 11 définissant la zone de captation.

La partie 13 formant zone de stockage et/ou évaporation peut être de forme annulaire continue angulairement, comme illustré à la figure 4, ou discontinue angulairement, comme illustré à la figure 5, chaque élément 14c de la structure de transfert 14 pouvant alors communiquer avec un unique élément respectif 13c formant réservoir.

Dans ce mode de réalisation, chaque élément 14c est isolé l'un de l'autre par un espace vide 17, ce dernier formant une barrière fluidique entre deux éléments 14c adjacents.

Dans la variante qui n'est pas selon l'invention illustrée à la figure 6, les éléments 14c de la structure de transfert 14 sont réalisés d'une seule pièce avec une partie centrale 14d qui se superpose à la partie absorbante éventuelle définissant la zone de captation et avec une partie périphérique 14e qui est reliée à la partie absorbante éventuelle formant zone de stockage et/ou évaporation et/ou qui définit une zone d'évaporation.

Dans les exemples des figures 4 à 6, les chemins de propagation fluidique sont définis par les éléments de transfert radiaux 14c, ceux-ci étant par exemple réalisés par découpe d'une feuille d'un matériau hydrophile drainant. Le liquide qui diffuse dans l'un des éléments 14c ne peut pas gagner les éléments adjacents, au moins dans la partie située entre la zone de captation et la zone de stockage et/ou d'évaporation, du fait de l'espace vide 17.

Dans les exemples des figures 1 à 6 qui viennent d'être décrits, comportant une structure de transfert 14 comportant plusieurs éléments reliant la zone de captation à une ou plusieurs parties formant zone de stockage et/ou évaporation, ces éléments sont disjoints et non superposés.

Comme illustré à la figure 7, les éléments de la structure de transfert 14 peuvent être définis par une seule couche d'un matériau drainant, comportant des parties hydrophobes 18 permettant de limiter, mieux d'empêcher, la circulation de liquide entre des parties hydrophiles 14f s'étendant entre les parties hydrophobes 18.

Les parties hydrophiles 14f peuvent s'étendre, comme illustré, sous la forme de bandes parallèles, se superposant partiellement à une extrémité avec une partie absorbante réceptrice 11 définissant la zone de captation et à l'autre extrémité avec une partie absorbante formant zone de stockage et/ou d'évaporation 13.

On a illustré à la figure 8 la possibilité pour la structure de transfert 14 de comporter deux éléments 14g qui se superposent au moins partiellement. Chaque élément 14g est recouvert supérieurement d'une couche barrière 19, imperméable à l'eau, de telle sorte que le liquide qui diffuse dans l'élément 14g ne puisse pas gagner l'élément de transfert 14g qui le recouvre.

Une superposition des éléments 14g peut permettre d'accroître le débit du liquide qui diffuse dans la structure de transfert, pour un même encombrement sur la peau.

Dans l'exemple qui n'est pas selon l'invention illustré sur les figures 9 et 10, l'article est réalisé d'une seule pièce dans une feuille d'un même matériau drainant.

L'article comporte une zone de captation 14d centrale et une zone de stockage et/ou d'évaporation périphérique 14e, reliées entre elles par des zones de transfert 14c. Les différentes zones sont réalisées par traitement localisé 20 de la feuille de matériau initial, par exemple un traitement hydrophobe appliqué à un matériau initial hydrophile afin de définir des zones, hachurées sur la figure, où le fluide ne peut pas se propager comme illustré sur la figure 9. Dans un autre exemple, les zones sont définies suite à un traitement hydrophile appliqué à un matériau initial hydrophobe, afin de définir les zones, hachurées sur la figure, où le fluide peut diffuser, comme illustré sur la figure 10.

L'invention n'est pas limitée aux exemples illustrés.

En particulier, on peut réaliser la structure de transfert avec d'autres formes encore.

L'expression « comportant un » doit se comprendre comme étant synonyme de « comportant au moins un ».

## Revendications

1. Article (10) ayant des propriétés d'absorption d'un fluide corporel, comprenant une structure de transfert (14) définissant au moins trois chemins de propagation fluidique (14a, 14b, 14c) depuis une partie absorbante réceptrice définissant une zone de captation dudit fluide, vers au moins une partie absorbante formant zone de stockage et/ou d'évaporation, chaque chemin de propagation fluidique (14a, 14b, 14c) étant délimité par une barrière fluidique (17, 18, 19), la barrière fluidique s'opposant au passage de liquide d'un chemin à un autre et s'étendant entre deux chemins de propagations fluidiques adjacents, les chemins de propagation fluidique étant définis par un matériau comportant des fibres absorbantes, la partie absorbante formant zone de stockage et/ou d'évaporation et la partie absorbante réceptrice étant chacune définie par un matériau ayant une capacité d'absorption du liquide supérieure à celle de la structure de transfert, la barrière fluidique comportant un espace libre (17) et/ou un matériau hydrophobe (18, 19).

2. Article selon la revendication 1, lesdits chemins étant définis par des éléments distincts (14a ; 14b ; 14c ; 14g) d'une structure de transfert (14).

3. Article selon la revendication 1 ou 2, les chemins de propagation fluidique étant définis par des éléments (14c) disposés avec un décalage angulaire autour d'un centre de l'article, notamment étant équi-angulairement répartis.

4. Article selon l'une quelconque des revendications précédentes, les chemins de propagation fluidique étant définis par des éléments (14g) séparés par une couche (19) d'un matériau imperméable à l'eau.

5. Article selon la revendication 1 ou 3 et 4, les chemins de propagation fluidique étant sensiblement parallèles entre eux

6. Article selon la revendication précédente, comportant deux ensembles de chemins de propagation fluidique entre une zone de captation commune de fluide et deux zones respectives de stockage et/ou d'évaporation, chaque zone de stockage et/ou d'évaporation étant propre à l'un des deux ensembles.

7. Article selon l'une quelconque des revendications 1 à 3, la zone de stockage et/ou d'évaporation entourant complètement la zone de captation, notamment lui étant concentrique.

8. Article selon l'une quelconque des revendications 1 à 4, les chemins de propagation fluidique débouchant chacun à deux extrémités opposées sur des parties absorbantes respectives formant zone de stockage et/ou d'évaporation.

9. Article selon l'une quelconque des revendications précédentes, les chemins de propagation fluidique étant définis par des éléments se superposant au moins partiellement.

10. Article selon l'une quelconque des revendications précédentes, constituant un pansement.

## Patentansprüche

1. Artikel (10) mit Eigenschaften zur Absorption einer Körperflüssigkeit, der eine Transportstruktur (14) umfasst, die mindestens drei Flüssigkeitsausbreitungsstrecken (14a, 14b, 14c) von einem aufnehmenden absorbierenden Teil aus definiert, der einen Auffangbereich für die Flüssigkeit definiert, hin zu mindestens einem absorbierenden Teil, der einen Speicher- und/oder Verdunstungsbereich bildet, wobei jede Flüssigkeitsausbreitungsstrecke (14a, 14b, 14c) von einer Flüssigkeitssperre (17, 18, 19) begrenzt wird, wobei die Flüssigkeitssperre das Fließen der Flüssigkeit von einer Strecke zur anderen verhindert und zwischen zwei benachbarten Flüssigkeitsausbreitungsstrecken verläuft, wobei die Flüssigkeitsausbreitungsstrecken durch ein Material definiert sind, das absorbierende Fasern aufweist, wobei der absorbierende Teil, der einen Speicher- und/oder Verdunstungsbereich bildet, und der aufnehmende absorbierende Teil jeweils durch ein Material definiert sind, das ein Flüssigkeitsabsorptionsvermögen aufweist, das höher ist als das der Transportstruktur, wobei die Flüssigkeitssperre einen Leerraum (17) und/oder ein hydrophobes Material (18, 19) aufweist.

2. Artikel nach Anspruch 1, wobei die Strecken durch einzelne Elemente (14a; 14b; 14c; 14g) einer Transportstruktur (14) definiert sind.

3. Artikel nach Anspruch 1 oder 2, wobei die Flüssigkeitsausbreitungsstrecken durch Elemente (14c) definiert sind, die winkelversetzt um einen Artikelmittelpunkt angeordnet sind, insbesondere mit demselben Winkel verteilt sind.

4. Artikel nach einem der vorhergehenden Ansprüche, wobei die Flüssigkeitsausbreitungsstrecken durch Elemente (14g) definiert sind, die durch eine Schicht (19) aus einem wasserundurchlässigen Material getrennt sind.

5. Artikel nach Anspruch 1 oder 3 und 4, wobei die Flüssigkeitsausbreitungsstrecken im Wesentlichen parallel zueinander verlaufen.

6. Artikel nach dem vorhergehenden Anspruch, der zwei Einheiten von Flüssigkeitsausbreitungsstrecken zwischen einem gemeinsamen Flüssigkeitsauffangbereich und zwei entsprechenden Speicher- und/oder Verdunstungsbereichen aufweist, wobei jeder Speicher- und/oder Verdunstungsbereich zu einer der beiden Einheiten gehört.

7. Artikel nach einem der Ansprüche 1 bis 3, wobei der Speicher- und/oder Verdunstungsbereich den Auffangbereich vollständig umgibt, insbesondere konzentrisch dazu ist.

8. Artikel nach einem der Ansprüche 1 bis 4, wobei die Flüssigkeitsausbreitungsstrecken jeweils an zwei gegenüberliegenden Enden zu den entsprechenden absorbierenden Teilen hin offen sind, die einen Speicher- und/oder Verdunstungsbereich bilden.

9. Artikel nach einem der vorhergehenden Ansprüche, wobei die Flüssigkeitsausbreitungsstrecken durch Elemente definiert sind, die zumindest teilweise übereinanderliegen.

10. Artikel nach einem der vorhergehenden Ansprüche, der einen Verband darstellt.

## Claims

1. An article (10) having bodily-fluid absorption properties, comprising a transfer structure (14) defining at least three fluidic-propagation paths (14a, 14b, 14c) leading from a zone in which said fluid is collected to at least one retention and/or evaporation zone, each fluidic-propagation path (14a, 14b, 14c) being delimited by a fluidic barrier (17, 18, 19), the fluidic barrier opposing the passage of liquid from one path to another and extending between two adjacent fluidic-propagation paths, the fluidic-propagation paths being defined by a material containing absorbent fibers, the at least one retention and/or evaporation zone and the zone in which the fluid is collected being each defined by a material having a collecting capacity of the fluid that is superior to the collecting capacity of the fluid of the transfer structure, the fluidic barrier comprising an empty space (17) and/or a hydrophobic material (18, 19).

2. The article as claimed in claim 1, said paths being defined by distinct elements (14a; 14b; 14c; 14g) of a transfer structure (14).

3. The article as claimed in any one of the preceding claims, the fluidic-propagation paths being defined by elements (14c) arranged with an angular offset about a center of the article, notably being evenly angularly distributed.

4. The article as claimed in any one of the preceding claims, the fluidic-propagation paths being defined by elements (14g) that are separated by a layer (19) of a material impermeable to water.

5. The article claimed in any one of claims 1 or 3 and 4, the fluidic-propagation paths being substantially parallel to one another.

6. The article as claimed in the preceding claim, comprising two sets of fluidic-propagation paths leading between a common fluid collection zone and two respective retention and/or evaporation zones, each retention and/or evaporation zone being specific to one of the two sets.

7. The article as claimed in any one of claims 1 to 3, the retention and/or evaporation zone completely surrounding the collection zone, notably being concentric therewith.

8. The article as claimed in any one of claims 1 to 4, the fluidic-propagation paths each opening at two opposite ends onto respective absorbent parts forming retention and/or evaporation zones.

9. The article as claimed in any one of the preceding claims, the fluidic-propagation paths being defined by elements that are at least partially superposed.

10. The article as claimed in any one of the preceding claims, constituting a dressing.
